# EUROPEAN PATENT SPECIFICATION

(11) **EP 2 774 630 B1**
(45) Date of publication and mention of the grant of the patent: **05.07.2017**
(21) Application number: 11875085.0
(22) Date of filing: 14.12.2011
(51) Int. Cl.: A61F 2/24, A61L 27/36

(54) **METHOD FOR PREPARING EDGE-RIGIDIZED ARTIFICIAL BIOLOGICAL VALVE**
VERFAHREN ZUR HERSTELLUNG EINES RANDVERSTEIFTEN KÜNSTLICHEN BIOLOGISCHEN KLAPPE
PROCÉDÉ POUR LA PRÉPARATION DE VALVULE BIOLOGIQUE ARTIFICIELLE RIGIDIFIÉE SUR LES BORDS

(30) Priority: 01.11.2011 CN 201110339534
(43) Date of publication of application: 10.09.2014
(73) Proprietor: Shanghai MicroPort CardioFlow Medtech Co., Ltd., Shanghai 201203 (CN)
(72) Inventor: CHEN, Dakai, Shanghai 201203 (CN); LI, Yu, Shanghai 201203 (CN); TIAN, Cong, Shanghai 201203 (CN); FANG, Yuan, Shanghai 201203 (CN); DONG, Jiaoming, Shanghai 201203 (CN); CHEN, Cheng, Shanghai 201203 (CN); CHENG, Xiulan, Shanghai 201203 (CN); CHEN, Guoming, Shanghai 201203 (CN); YUE, Chengyun, Shanghai 201203 (CN); LUO, Qiyi, Shanghai 201203 (CN)
(74) Representative: Dantz, Jan Henning
(86) International application number: PCT/CN2011/083987
(87) International publication number: WO 2013/063842

(56) References cited:
- WO-A1-97/46266
- WO-A2-2004/047620
- CN-A- 101 184 516
- US-A- 4 648 881
- US-A1- 2003 226 208
- US-A1- 2006 217 804
- US-B1- 6 561 970

## Description

### Technical field

The present invention relates to the field of medical devices. In particular, the present invention relates to a method for preparing an edge-rigidized artificial biological valve (film).

### Background Art

The development of an artificial biological (cardiac) valve has been one of the research topics in the field of cardiac surgery. An ideal artificial biological valve should meet the following criteria: a good durability and biocompatibility, a hemodynamic performance which is close to that exhibited by a physiological valve, minimal damage to blood cells, easy to implant, resistance to infection, non-immunogenicity, etc.

A minimally invasive artificial biological valve (film) includes a balloon-expandable biological valve and a self-expandable biological valve.

The balloon-expandable biological valve is an artificial biological valve that is fixed on a plastically deformable stent. The valve is fixed to the balloon by radially compressing the stent. After the valve is delivered into the aortic valve by minimally invasive intervention, the stent is expanded and fixed by pressurizing the balloon. Such a balloon-expandable biological valve has the following disadvantage and problem: during the processes of stent compression and balloon expansion, the leaflet tissue structure of the biological valve will suffer great damage, which severely affects the service life of the cardiac valve after implantation. Besides, the size of the stent for the cardiac valve is decided by the diameter of the balloon. If the selected size is too small, the valve is at the risk of looseness or displacement, and thereby a secondary balloon expansion has to be performed; and if the selected size is too large, there is a risk of tearing the aortic annulus, which results in the occurrence of additional complications. With respect to such a cardiac valve, the balloon cannot be reset once it is expanded, and the improper placement of the valve will endanger the patient's life on the spot; and once the cardiac valve is implanted, it cannot be retrieved and has to be surgically replaced if a problem arises.

The self-expandable artificial cardiac valve is a biological valve that is fixed on a self-expandable nitinol stent. The stent is placed into a catheter of a carrier and released after being delivered into the aortic valve by minimally invasive intervention. The sent is then fixed to the aortic annulus by virtue of its own structure. Such a self-expandable biological valve has the following disadvantages and problems: the stent is too long and has a nonideal opening design, which is likely to affect the hemodynamics at the left and right coronary ostia and results in cardiac dysfunction; the cardiac valve which is released improperly cannot be reset and will endanger the patient's life; and once the cardiac valve is implanted, it cannot be retrieved and has to be surgically replaced if a problem arises.

The current artificial biological valve cannot completely achieve the purpose of being freely released. In the release process of an artificial biological valve, the bloodstream at the center would readily impact on the edge of the artificial biological valve and thereby hinder the subsequent release process. Additionally, as observed from the calcification phenomenon of the pericardial material and from the damage phenomenon occurred during the aging process, the edge of the artificial biological valve is usually the most vulnerable site to calcification and aging which also seriously affect the use of the artificial biological valve. The main problem to be solved in the process of developing an artificial biological valve is how to ensure that a biological valve is freely released and less prone to edge calcification and damage in use, and that the biological valve is structurally closer to the valve in a normal body.

In Reference 1 [Hsing-Wen Sung, Yen Chang, Chi-Tung Chiu, Chiun-Nan Chen, Huang-Chien Liang. Mechanical properties of a porcine aortic valve fixed with a naturally occurring crosslinking agent. Biomaterials, 1999, 20: 1759-1772], the collagen fibers in an artificial biological valve are treated with genipin and compared with those treated by the traditional glutaraldehyde method. It is found that genipin has a better anti-calcification effect compared with the traditional glutaraldehyde treatment. However, no analysis is performed for the edge breakage of the artificial biological valve.

In Reference 2 [Peter Angele, Jochen Abke, Richard Kujat, Hubert Faltermeier, Detlef Schumann, Michael Nerlich, Bernd Kinner, Carsten Englert, Zbigniew Ruszczak, Robert Mehrl, Rainer Mueller. Influence of different collagen species on physico-chemical properties of crosslinked collagen matrices. Biomaterials, 2004, 25: 2831-2841], the collagen fibers in an artificial biological valve are treated with carbodiimide and investigated for the mechanical property and swelling property. However, no treatment and research are performed for the overall durability of the artificial biological valve.

Chinese patent application No. 200680018417.4 (USA Edwards Lifesciences Corporation) discloses treatment of bioprosthetic tissues to mitigate post implantation calcification. In this application, a heat-treated or pH-adjusted glutaraldehyde solution is used to pretreat the bioprosthetic tissue, and then a blocking agent and a reducing agent are used in combination to construct a calcification-resistant artificial biological valve.

Chinese patent application No. 92100096.0 (Beijing Fu Wai Hospital) discloses a method for chemically modifying a heterogeneous biological valve, wherein hydroxy-chromium and glutaraldehyde are used in combination to construct a calcification-resistant artificial biological valve.

US 2006/217804 describes a method to reduce thrombogenicity and post implantation calcification of a tissue, said method comprising heating tissue in an aqueous glutaraldehyde solution, contacting it with a solution of a blocking agent to block free amine groups, and treating the tissue with a reducing agent to reduce aldehyde and carboxylic acid groups.

### Summary of the Invention

The object of the present invention is to provide a method for preparing an edge-rigidized artificial biological valve as defined in independent claim 1, so as to overcome the above defects in the prior art.

The method of the present invention comprises: first, as a pretreatment step, soaking an artificial biological valve in a glutaraldehyde solution, wherein the artificial biological valve can be partly fixed with glutaraldehyde before, after, or during contact with the glutaraldehyde solution; and then blocking the free aldehyde groups, carboxyl groups, amino groups, hydroxyl groups and carbonyl groups formed on the artificial biological valve pretreated with glutaraldehyde by a reducing agent and tannin extract.

Without being restricted by any existing theory, the edge-rigidization of the artificial biological valve of the present invention is enhanced presumably by the following mechanism: glutaraldehyde reacts chemically with the collagen in the artificial biological valve, primarily by the condensation reaction with the ε-amino groups of lysine in the collagen to generate a strong cross linkage, and also by the formation of an acetal with the hydroxy groups of hydroxyproline to produce a strong cross linkage. In addition to the above reactions, alcoholization reaction can also occur. By the above three kinds of reaction, glutaraldehyde allows for the intramolecular and intermolecular crosslinking of the collagen molecules, and thereby enables an enhanced structural strength. A reducing agent such as sodium borohydride can reduce the free aldehyde groups produced during the process of treating and modifying the artificial biological valve with the glutaraldehyde solution, so as to reduce the number of aldehyde groups on the surface of the artificial biological valve and improve the calcification resistance of the artificial biological valve. The tannin extract, as a modifier of natural compounds, substantially consists of tannins, non-tannins, simple phenols, organic acids, etc. In PBS, Hank's or D-Hank's buffer solution (for the composition, see Tables 3-5 below), polyphenolic carboxylic acids and polyols in the hydrolyzed tannins will bond to the aldehyde groups, carboxyl groups, amino groups, hydroxyl groups, carbonyl groups and the like on the surface of the artificial artificial biological valve through an ester bond or a glycoside bond, to form complex compounds. Meanwhile, polycondensate of flavanols in the condensed tannins will undergo polycondensation with acids under heat and fill the surface of the artificial biological valve. The addition of a sulfite can reduce the content of insolubles in the PBS, Hank's or D-Hank's buffer solution containing tannin extract, enhance the cold solubility, increase the penetration rate and lighten the color, so as to obtain an artificial biological valve with a good interface performance. The sulfite includes one or more of potassium sulfite, potassium bisulfite, sodium sulfite, sodium bisulfite, zinc sulfite and ammonium sulfite in the concentration range of 0.1-30%.

In particular, the present invention provides a method for preparing an edge-rigidized artificial biological valve comprising the following steps:
after sufficiently rinsing a fresh material of artificial biological valve with a PBS, Hank's or D-Hank's buffer solution (pH 7.0-7.8), selecting the artificial biological valve with a uniform thickness and a consistent fiber orientation, cutting the selected artificial biological valve into patches with a size of 1-60 cm², placing and fixing the patches in a glutaraldehyde complex buffer solution for pretreatment for 1 to 25 days, and then washing the artificial biological valve with double distilled water to wash out the glutaraldehyde;
placing and modifying the artificial biological valve pretreated with glutaraldehyde in a PBS, Hank's or D-Hank's buffer solution containing a reducing agent for 1 to 20 hours; and
placing the artificial biological valve treated with the solution containing the reducing agent into a PBS, Hank's or D-Hank's buffer solution containing tannin extract for 30 to 60 days, followed by removing, sufficiently rising with a PBS buffer solution, and washing with double distilled water prior to storage in double distilled water.

In the present invention, the reducing agent used is one or more species selected from the group consisting of sodium borohydride, lithium aluminum hydride, potassium borohydride, sodium thioborohydride, lithium tri-sec-butylborohydride, aluminum isopropoxide, zinc powder and magnesium powder.

In the present invention, the glutaraldehyde complex buffer solution used is one or more species selected from the group consisting of glutaraldehyde/hydroxyethylpiperazinyl ethanesulfonic acid complex buffer solution, glutaraldehyde/anhydrous morpholino ethanesulfonic acid complex buffer solution, glutaraldehyde/Tris ethanesulfonic acid complex buffer solution, and glutaraldehyde/tris(hydroxymethyl)aminomethane complex buffer solution, wherein glutaraldehyde is at the concentration of 0.1-10%, hydroxyethylpiperazinyl ethanesulfonic acid is at the concentration of 0.1-10%, anhydrous morpholino ethanesulfonic acid is at the concentration of 0.1-10%, Tris ethanesulfonic acid is at the concentration of 0.1-10%, and tris(hydroxymethyl)aminomethane is at the concentration of 0.1-10%, and the glutaraldehyde complex buffer solution has a pH range of 5.0-7.8.

In the present invention, the tannin extract used is one or more species selected from the group consisting of myrica tannin extract, wattle tannin extract, gallnut tannin extract, oak tannin extract, larch tannin extract, citrus tannin extract, *Rhizoma Arisaematis Calcarei* tannin extract, quebracho tannin extract, mangrove tannin extract, *Terminalia chebula Retz .* tannin extract, chestnut tannin extract, piling wood tannin extract, valonia tannin extract, betel nut extract, gooseberry tannin extract, black wattle tannin extract, valonea tannin extract, Acacia mangium tannin extract, etc.

The method of the present invention provides an enhanced edge rigidity of the artificial biological valve, and thereby improves the long-term stability and durability of the artificial biological valve.

### Description of Figures

In order to more clearly describe the technical solutions of the present invention, brief description is made below in conjunction with the accompanying drawings. Apparently, these drawings are merely some of the specific embodiments described in the present application. The technical solutions of the present invention include, but are not limited to these drawings.

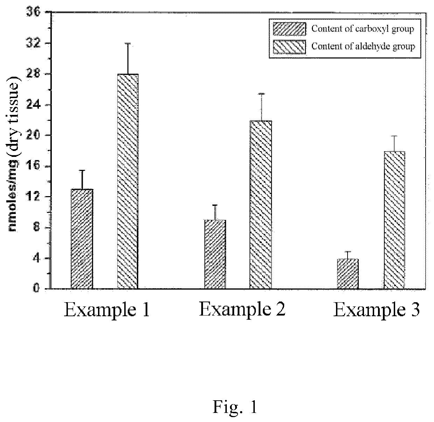

Fig. 1 shows the contents of aldehyde group and carboxyl group in the artificial biological valve treated according to Example 1, Example 2 and Example 3.

### Embodiments

For a further understanding of the present invention, the preferred embodiments of the present invention will be described in conjunction with following examples. The description is only illustrative of the features and advantages of the method of the present invention, and is not intended to limit the protection scope of the present invention.

### Example 1

An artificial biological valve is fixed in 0.625% glutaraldehyde/0.5% hydroxyethylpiperazinyl ethanesulfonic acid complex buffer solution (pH 7.4) for 3 days.

The artificial biological valve pretreated with glutaraldehyde is placed and modified in a PBS buffer solution (pH 6.8) containing 0.5% sodium borohydride for 5 hours.

The artificial biological valve pretreated with glutaraldehyde and treated with the solution containing sodium borohydride is placed and treated in a PBS solution (pH 6.8) containing 0.5% myrica tannin extract for 2 days.

### Example 2

An artificial biological valve is fixed in 0.625% glutaraldehyde/0.5% anhydrous morpholino ethanesulfonic acid complex buffer solution (pH 7.4) for 15 days.

The artificial biological valve pretreated with glutaraldehyde is placed and modified in a PBS buffer solution (pH 6.8) containing 0.5% sodium borohydride for 10 hours.

The artificial biological valve pretreated with glutaraldehyde and treated with the solution containing sodium borohydride is placed and treated in a PBS buffer solution (pH 6.8) containing 0.5% wattle tannin extract for 3 days.

### Example 3

An artificial biological valve is fixed in 0.625% glutaraldehyde/0.5% tris(hydroxymethyl)aminomethane complex buffer solution (pH 7.4) for 20 days.

The artificial biological valve pretreated with glutaraldehyde is placed and modified in a Hank's buffer solution (pH 6.8) containing 1% sodium borohydride for 20 hours.

The artificial biological valve pretreated with glutaraldehyde and treated with the solution containing sodium borohydride is placed and treated in a Hank's buffer solution (pH 6.8) containing 1% quebracho tannin extract for 7 days.

### Example 4

The methods for measuring tensile strength, bending strength, residual dry weight and percentage of tearing points are as follows:

### Tensile Test

Test Instrument: Instron 5543 Model tensile testing machine
Test Condition: room temperature, PBS water bath
Test Method: An artificial biological valve is cut into a test strip of 5 mm × 50 mm; the two ends of the test strip are fixed to clamps wherein the upper and lower clamps are separated by a distance of 25 mm; after fixation, the strip is preloaded under the following preloading conditions: the rate of 60 mN/min, the maximum value of 1 mN, 3 cycles; upon the completion of presetting, the specimen is loaded with a stress at the loading rate of 25 mm/min along the fiber arrangement direction; and corresponding indexes are measured to calculate the tensile strength of the material.

### Bending test

Test Instrument: Pulse Fluid Impact testing machine
Test Condition: room temperature, PBS water bath
Test Method: An artificial biological valve is cut into a test strip of 15 mm × 60 mm; one end of the test strip is fixed to a clamp; the center of the test strip is disposed 50 mm away from the emission port of the pulse fluid, wherein the pulse fluid pressure is 20 mmHg with 3 cycles; and corresponding indexes are measured to calculate the bending strength of the material.

### Residual Dry Weight Test

Instrument: -80°C refrigerator, vacuum freeze dryer
Procedure: A specimen is pre-frozen in the -80°C refrigerator for 1 h, lyophilized in the vacuum freeze dryer for 24 h, removed and weighed so as to determine the dry weight.

### Tearing Point Test

Test Instrument: High pressure fluid testing machine
Test Condition: room temperature, PBS water bath
Test Method: An artificial biological valve is stitched on a nitinol stent, and the assembly is fixed to a clamp, wherein the fluid pressure is set at 300 mmHg, the number of cycles is 75 cycles per minute with a total number of 200 million. At the end, corresponding indexes are measured to calculate the tearing points of the artificial biological valve in the assembly.

The measurement results are shown in Tables 1-2 and Fig. 1.

**Table 1: Comparison of the mechanical properties of the artificial biological valves treated according to Example 1, Example 2 and Example 3**

| Example | Tensile strength (MPa) | Bending strength (mN/mm²) |
|---|---|---|
| Example 1 | 10.07 | 75 |
| Example 2 | 11.45 | 108 |
| Example 3 | 14.69 | 146 |

**Table 2: The residual dry weight and percentage of tearing points of the artificial biological valves treated according to Example 1, Example 2 and Example 3, as measured 35 days after the 300 mmHg high pressure fluid test**

| Example | Initial dry weight (mg) | Residual dry weight (mg) | Percentage of tearing point (%) |
|---|---|---|---|
| Example 1 | 830.02 | 787.56 | 14.50 |
| Example 2 | 832.97 | 805.89 | 5.78 |
| Example 3 | 831.45 | 827.23 | 1.24 |

It can be clearly seen from Fig. 1, Table 1 and Table 2 that the artificial biological valve prepared according to the method of the present invention has a reasonable distribution of the groups on the surface, an excellent mechanical property, and a good measurement result obtained under high pressure fluid.

**Table 3: The ingredients in the PBS buffer solution (g/L)**

| Ingredient | PBS |
|---|---|
| NaCl | 8 |
| KCl | 0.2 |
| Na₂HPO₄ | 1.42 |
| KH₂PO₄ | 0.27 |

**Table 4: The ingredients in the Hank's buffer solution (g/L)**

| Ingredient | Hank's |
|---|---|
| CaCl₂ (anhydrous) | 0.14 |
| KCl | 0.4 |
| KH₂PO₄ | 0.06 |
| MgCl₂·6H₂O | 0.10 |
| MgSO₄·7H₂O | 0.10 |
| NaCl | 8.0 |
| NaHCO₃ | 0.35 |
| Na₂HPO₄·7H₂O | 0.09 |
| D-glucose | 1.0 |
| Phenol red (0.1 %) | 1 mL |

**Table 5: The ingredients in the D-Hank's buffer solution (g/L)**

| Ingredient | Hank's |
|---|---|
| KCl | 0.4 |
| KH₂PO₄ | 0.06 |
| NaCl | 8.0 |
| NaHCO₃ | 0.35 |
| Na₂HPO₄·12H₂O | 0.132 |
| D-glucose | 1.0 |
| Phenol red (0.1 %) | 1 mL |

The method of the present invention can improve the mechanical property and calcification resistance of an artificial biological valve during the chemical modification process, and meanwhile, improve the overall durability of the artificial biological valve.

## Claims

1. A method for preparing an edge-rigidized artificial biological valve comprising: first soaking an artificial biological valve in a glutaraldehyde complex buffer solution for pretreatment, wherein the artificial biological valve can be partly fixed with glutaraldehyde before, after, or during contact with the glutaraldehyde complex buffer solution; and then blocking the free aldehyde groups, carboxyl groups, amino groups, hydroxyl groups and carbonyl groups formed on the artificial biological valve pretreated with glutaraldehyde by a reducing agent and tannin extract sequentially.

2. The method according to claim 1, comprising the following steps:
after sufficiently rinsing a fresh material of artificial biological valve with a PBS, Hank's or D-Hank's buffer solution at pH 7.0-7.8, selecting the artificial biological valve with a uniform thickness and a consistent fiber orientation, cutting the selected artificial biological valve into patches with a size of 1-60 cm², placing and fixing the patches in a glutaraldehyde complex buffer solution for pretreatment for 1 to 25 days, and then washing the artificial biological valve with double distilled water to wash out the glutaraldehyde;
placing and modifying the artificial biological valve pretreated with glutaraldehyde in a PBS, Hank's or D-Hank's buffer solution containing a reducing agent for 1 to 20 hours; and
placing the artificial biological valve treated with the solution containing the reducing agent into a PBS, Hank's or D-Hank's buffer solution containing tannin extract for 30 to 60 days, followed by removing, sufficiently rising with a PBS solution, and washing with double distilled water prior to storage in double distilled water.

3. The method according to claim 1 or 2, wherein the reducing agent is one or more species selected from the group consisting of sodium borohydride, lithium aluminum hydride, potassium borohydride, sodium thioborohydride, lithium tri-sec-butylborohydride, aluminum isopropoxide, zinc powder and magnesium powder.

4. The method according to claim 2, wherein the glutaraldehyde complex buffer solution is one or more species selected from the group consisting of glutaraldehyde/hydroxyethylpiperazinyl ethanesulfonic acid complex buffer solution, glutaraldehyde/anhydrous morpholino ethanesulfonic acid complex buffer solution, glutaraldehyde/Tris ethanesulfonic acid complex buffer solution, and glutaraldehyde/tris(hydroxymethyl)aminomethane complex buffer solution.

5. The method according to claim 4, wherein glutaraldehyde is at the concentration of 0.1-10%, hydroxyethylpiperazinyl ethanesulfonic acid is at the concentration of 0.1-10%, anhydrous morpholino ethanesulfonic acid is at the concentration of 0.1-10%, Tris ethanesulfonic acid is at the concentration of 0.1-10%, and tris(hydroxymethyl)aminomethane is at the concentration of 0.1-10%, and the glutaraldehyde complex buffer solution has a pH range of 5.0-7.8.

6. The method according to any one of claims 1-5, wherein the tannin extract is one or more species selected from the group consisting of myrica tannin extract, wattle tannin extract, gallnut tannin extract, oak tannin extract, larch tannin extract, citrus tannin extract, *Rhizoma Arisaematis Calcarei* tannin extract, quebracho tannin extract, mangrove tannin extract, *Terminalia chebula Retz.* tannin extract, chestnut tannin extract, piling wood tannin extract, valonia tannin extract, betel nut extract, gooseberry tannin extract, black wattle tannin extract, valonea tannin extract, and Acacia mangium tannin extract.

7. The method according to claim 2, wherein a sulfite is added into the PBS, Hank's or D-Hank's buffer solution containing tannin extract.

8. The method according to claim 7, wherein the sulfite is one or more species selected from the group consisting of potassium sulfite, potassium bisulfite, sodium sulfite, sodium bisulfite, zinc sulfite and ammonium sulfite.

9. The method according to claim 7 or 8, wherein the sulfite is at the concentration of 0.1-30%.

## Patentansprüche

1. Verfahren zur Herstellung einer randversteiften künstlichen biologischen Klappe, umfassend: zunächst Eintauchen einer künstlichen biologischen Klappe in eine Glutaraldehydkomplex-Pufferlösung zur Vorbehandlung, wobei die künstliche biologische Klappe vor, nach oder während des Kontakts mit der Glutaraldehydkomplex-Pufferlösung teilweise mit Glutaraldehyd fixiert werden kann; und anschließend nacheinander erfolgendes Blockieren der freien Aldehydgruppen, Carboxylgruppen, Aminogruppen, Hydroxylgruppen und Carbonylgruppen, die sich auf der mit Glutaraldehyd vorbehandelten künstlichen biologischen Klappe gebildet haben, durch ein Reduktionsmittel und Tanninextrakt.

2. Verfahren nach Anspruch 1, umfassend die folgenden Schritte:
nach ausreichendem Spülen eines frischen Materials für die künstliche biologische Klappe mit phosphatgepufferter Salzlösung, Hanks- oder D-Hanks-Pufferlösung mit einem pH von 7,0 bis 7,8 Auswählen der künstlichen biologischen Klappe mit einer gleichmäßigen Dicke und einer einheitlichen Faserausrichtung, Schneiden der ausgewählten künstlichen biologischen Klappe in Stücke mit einer Größe von 1 bis 60 cm2, Anordnen und Fixieren der Stücke in einer Glutaraldehydkomplex-Pufferlösung zur Vorbehandlung während 1 bis 25 Tagen, und anschließend Waschen der künstlichen biologischen Klappe mit doppelt destilliertem Wasser, um das Glutaraldehyd auszuwaschen;
Anordnen und Modifizieren der mit Glutaraldehyd vorbehandelten künstlichen biologischen Klappe in phosphatgepufferter Salzlösung, Hanks- oder D-Hanks-Pufferlösung, die ein Reduktionsmittel enthält, während 1 bis 20 Stunden; und
Anordnen der künstlichen biologischen Klappe, die mit der das Reduktionsmittel enthaltenden Lösung behandelt wurde, in einer Tanninextrakt enthaltenden phosphatgepufferten Salzlösung, Hanks- oder D-Hanks-Pufferlösung während 30 bis 60 Tagen, und anschließend Entnehmen, ausreichendes Spülen mit einer phosphatgepufferten Salzlösung und Waschen mit doppelt destilliertem Wasser vor dem Aufbewahren in doppelt destilliertem Wasser.

3. Verfahren nach Anspruch 1 oder 2, wobei es sich bei dem Reduktionsmittel um eine oder mehrere Spezies handelt, die aus der Gruppe ausgewählt sind, die aus Natriumborhydrid, Lithiumaluminiumhydrid, Kaliumborhydrid, Natriumthioborhydrid, Lithium-tri-sec-butylborhydrid, Aluminiumisopropoxid, Zinkpulver und Magnesiumpulver besteht.

4. Verfahren nach Anspruch 2, wobei es sich bei der Glutaraldehydkomplex-Pufferlösung um eine oder mehrere Spezies handelt, die aus der Gruppe ausgewählt sind, die aus einer Pufferlösung aus einem Komplex aus Glutaraldehyd/Hydroxyethylpiperazinyl-ethansulfonsäure, einer Pufferlösung aus einem Komplex aus Glutaraldehyd/wasserfreier Morpholino-ethansulfonsäure, einer Pufferlösung aus einem Komplex aus Glutaraldehyd/Tris-ethansulfonsäure und einer Pufferlösung aus einem Komplex aus Glutaraldehyd/Tris(hydroxymethyl)aminomethan besteht.

5. Verfahren nach Anspruch 4, wobei Glutaraldehyd in einer Konzentration von 0,1 bis 10% vorliegt, Hydroxyethylpiperazinyl-ethansulfonsäure in einer Konzentration von 0,1 bis 10% vorliegt, die wasserfreie Morpholino-ethansulfonsäure in einer Konzentration von 0,1 bis 10% vorliegt, Tris-ethansulfonsäure in einer Konzentration von 0,1 bis 10% vorliegt und Tris(hydroxymethyl)aminomethan in einer Konzentration von 0,1 bis 10% vorliegt und die Glutaraldehydkomplex-Pufferlösung einen pH-Bereich von 5,0 bis 7,8 aufweist.

6. Verfahren nach einem der Ansprüche 1 bis 5, wobei es sich bei dem Tanninextrakt um eine oder mehrere Spezies handelt, die aus der Gruppe ausgewählt sind, die aus Myrica-Tanninextrakt, Akazien-Tanninextrakt, Gallapfel-Tanninextrakt, Eichen-Tanninextrakt, Lärchen-Tanninextrakt, Zitrusgewächs-Tanninextrakt, Rhizoma Arisaematis Calcarei-Tanninextrakt, Quebrachobaum-Tanninextrakt, Mangroven-Tanninextrakt, Terminalia chebula Retz-Tanninextrakt, Kastanien-Tanninextrakt, "Piling Wood"-Tanninextrakt, Walloneneichen-Tanninextrakt, Betelnuss-Extrakt, Stachelbeeren-Tanninextrakt, Schwarzholz-Akazien-Tanninextrakt, Valonea-Tanninextrakt und Acacia mangium-Tanninextrakt besteht.

7. Verfahren nach Anspruch 2, wobei ein Sulfit der Tanninextrakt enthaltenden phosphatgepufferten Salzlösung, Hanks- oder D-Hanks-Pufferlösung zugesetzt wird.

8. Verfahren nach Anspruch 7, wobei es sich bei dem Sulfit um eine oder mehrere Spezies handelt, die aus der Gruppe ausgewählt sind, die aus Kaliumsulfit, Kaliumbisulfit, Natriumsulfit, Natriumbisulfit, Zinksulfit und Ammoniumsulfit besteht.

9. Verfahren nach Anspruch 7 oder 8, wobei das Sulfit in einer Konzentration von 0,1 bis 30% vorliegt.

## Revendications

1. Procédé pour la préparation d'une valve artificielle biologique à bords raidis, comprenant : le prétraitement par trempage d'une valve biologique artificielle dans une solution tampon de complexe de glutaraldéhyde, la valve biologique artificielle pouvant être partiellement fixée avec du glutaraldéhyde avant, après ou pendant le contact avec la solution tampon de complexe de glutaraldéhyde, puis le blocage des groupes aldéhyde, des groupes carboxyle, des groupes amino, des groupes hydroxyle et des groupes carbonyle libres formés sur la valve biologique artificielle prétraitée avec le glutaraldéhyde, successivement avec un agent réducteur et avec un extrait de tanin.

2. Procédé selon la revendication 1, comprenant les étapes suivantes :
après rinçage suffisant d'un matériau neuf de valve biologique artificielle avec une solution de tampon PBS, de Hanks ou D-Hanks à pH 7,0 à 7,8, sélection de la valve biologique artificielle ayant une épaisseur uniforme et une orientation des fibres constante, découpe de la valve biologique artificielle choisie en pièces d'une taille de 1 à 60 cm², placement et fixation des pièces dans une solution tampon de complexe de glutaraldéhyde pour leur prétraitement pendant 1 à 25 jours, puis lavage de la valve biologique artificielle avec de l'eau bidistillée pour éliminer le glutaraldéhyde ;
placement et modification de la valve biologique artificielle prétraitée avec du glutaraldéhyde dans une solution de tampon PBS, de Hanks ou D-Hanks contenant un agent réducteur pendant 1 à 20 heures ; et
placement de la valve biologique artificielle traitée avec la solution contenant l'agent réducteur dans une solution de tampon PBS, de Hanks ou D-Hanks contenant un extrait de tanin pendant 30 à 60 jours, puis retrait de la valve, rinçage suffisant avec une solution de PBS et lavage avec de l'eau bidistillée avant stockage dans de l'eau bidistillée.

3. Procédé selon la revendication 1 ou 2, dans lequel l'agent réducteur est une ou plusieurs espèces choisies dans le groupe contenant le borohydrure de sodium, l'hydrure de lithium et d'aluminium, le borohydrure de potassium, le thioborohydrure de sodium, le tri-sec-butylborohydrure de lithium, l'isopropoxyde d'aluminium, la poudre de zinc et la poudre de magnésium.

4. Procédé selon la revendication 2, dans lequel la solution tampon de complexe de glutaraldéhyde est une ou plusieurs espèces choisies dans le groupe comprenant une solution tampon de complexe de glutaraldéhyde et d'acide hydroxyéthylpipérazinyl-éthanesulfonique, une solution tampon de complexe de glutaraldéhyde et d'acide morpholinoéthanesulfonique anhydre, une solution tampon de complexe de glutaraldéhyde et d'acide tris-éthanesulfonique et une solution tampon de glutaraldéhyde et de tris(hydroxyméthyl)aminométhane.

5. Procédé selon la revendication 4, dans lequel le glutaraldéhyde se trouve à une concentration de 0,1 à 10 %, l'acide hydroxyéthylpipérazinyl-éthanesulfonique à une concentration de 0,1 à 10 %, l'acide morpholino-éthanesulfonique anhydre à une concentration de 0,1 à 10 %, l'acide tris-éthanesulfonique à une concentration de 0,1 à 10 % et le tris(hydroxyméthyl)aminométhane à une concentration de 0,1 à 10 %, et la solution tampon de complexe de glutaraldéhyde a une plage de pH de 5,0 à 7,8.

6. Procédé selon l'une des revendications 1 à 5, dans lequel l'extrait de tanin est une ou plusieurs espèces choisies parmi le groupe comprenant l'extrait de tanin de myrica, l'extrait de tanin de mimosa, l'extrait de tanin de noix de galle, l'extrait de tanin de chêne, l'extrait de tanin d'épicéa, l'extrait de tanin de citrus, l'extrait de tanin de rhizome d'*Arisaema calcarei*, l'extrait de tanin de quebracho, l'extrait de tanin de palétuvier, l'extrait de tanin de *Terminalia chebula* Retz., l'extrait de tanin de marronnier, l'extrait de tanin de bois empilé (piling wood tannin extract), l'extrait de tanin de valonia (valonia tannin extract), l'extrait de tanin de noix d'arec, l'extrait de tanin de groseillier à maquereau, l'extrait de tanin d'acacia noir, l'extrait de tanin de valonée (valonea tannin extract) et l'extrait de tanin d'*Acacia mangium.*

7. Procédé selon la revendication 2, dans lequel un sulfite est ajouté à la solution de tampon PBS, de Hanks ou D-Hanks contenant l'extrait de tanin.

8. Procédé selon la revendication 7, dans lequel le sulfite est une ou plusieurs espèces choisies dans le groupe comprenant le sulfite de potassium, le bisulfite de potassium, le sulfite de sodium, le bisulfite de sodium, le sulfite de zinc et le sulfite d'ammonium.

9. Procédé selon la revendication 7 ou 8, dans lequel le sulfite se trouve à une concentration de 0,1 à 30 %.
